(19) Europäisches Patentamt
European Patent Office
Office européen des brevets

(11) Numéro de publication: **0 491 601 B1**

(12) **FASCICULE DE BREVET EUROPEEN**

(45) Date de publication du fascicule du brevet: **11.10.95**

(51) Int. Cl.[6]: **B41M 5/26**, C07C 15/16, C07C 25/18

(21) Numéro de dépôt: **91403367.5**

(22) Date de dépôt: **12.12.91**

(54) **Matériaux d'enregistrement sensibles à la chaleur.**

(30) Priorité: **19.12.90 FR 9015922**

(43) Date de publication de la demande: **24.06.92 Bulletin 92/26**

(45) Mention de la délivrance du brevet: **11.10.95 Bulletin 95/41**

(84) Etats contractants désignés: **AT BE CH DE DK ES FR GB GR IT LI LU NL SE**

(56) Documents cités:
**EP-A- 0 008 251**
**EP-A- 0 070 218**
**FR-A- 2 237 866**

**PATENT ABSTRACTS OF JAPAN vol. 9, no. 264 (M-423)(1987) 22 Octobre 1985; JP A 60 110 486 (HONSHIYUU SEISHI) 15.06.1985**

(73) Titulaire: **ELF ATOCHEM S.A.**
**4 & 8, Cours Michelet**
**La Défense 10**
**F-92800 Puteaux (FR)**

(72) Inventeur: **Commandeur, Raymond**
**Le Rocher,**
**Avenue de Vénaria**
**F-38220 Vizille (FR)**
Inventeur: **Sarron, Jean-Pierre**
**18, Rue du Général Colin**
**F-78400 Chatou (FR)**

**EP 0 491 601 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne l'application d'une famille de polyphénylméthanes substitués dans les matériaux d'enregistrement sensibles à la chaleur et ces matériaux en eux-mêmes. Un exemple de ces matériaux est le papier thermique qu'on utilise dans des imprimantes de calculateurs, ou dans les téléfax. Ces matériaux d'enregistrement sensibles à la chaleur sont constitués d'un substrat, pouvant être du papier, sur lequel on a déposé un précurseur de colorant et un développeur. Sous l'action de la chaleur le précurseur et le développeur produisent une couleur.

Selon l'art antérieur EP 164417 on a perfectionné ces matériaux en ajoutant au précurseur et au développeur un benzylbiphényl ou un terphényl éventuellement substitué et pouvant aussi avoir été hydrogéné.

D'après cet art antérieur on obtient ainsi une meilleure qualité tout en ayant une plus grande vitesse d'impression.

Le brevet US 4 742 042 décrit des matériaux similaires à l'art antérieur précédent mais dans lequel le benzylbiphényl peut être remplacé par des esters ou des diesters.

Le biphényl et les terphényles sont coûteux à fabriquer parce qu'il faut des procédés à haute température. D'après ULLMAN'S ENCYCLOPEDIA OF INDUSTRIAL CHEMISTRY 5e édition - Vol A 13 - pages 261-265, le biphényl est produit par hydrodealkylation du toluène à 700 °C et les terphényles par déshydrocondensation du benzène entre 700 et 850 °C.

La demande de brevet FR-A-2237866 décrit un procédé de préparation d'o-benzyltoluènes qui consiste à faire réagir un halogénure d'o-xylyle avec un benzène éventuellement substitué en présence de trifluorure de bore ou d'un dérivé du trifluorure de bore et en présence d'un acide oxygéné formant avec ces composés du bore un composé d'addition.

On a maintenant trouvé des produits utiles dans ces matériaux d'enregistrement sensibles à la chaleur et qui sont faciles à fabriquer. Ces produits sont des polyphénylméthanes substitués. La présente invention concerne des matériaux d'enregistrement sensibles à la chaleur constitués d'un substrat ayant une couche contenant un précurseur de colorant et un développeur sensibles à la chaleur la dite couche contenant aussi un polyphénylméthane de formule A1

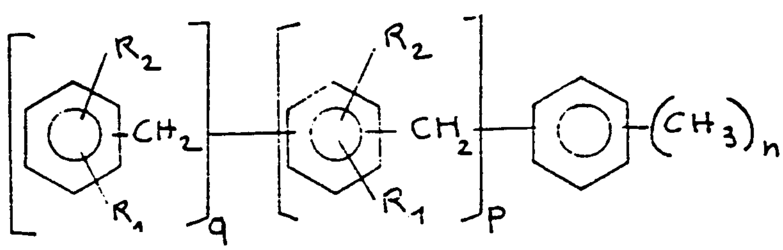

(A1)

dans laquelle :

$R_1$, $R_2$ identiques ou différents sont choisis parmi les halogènes, $NO_2$, CN, $OCH_3$, H ou un alkyle ayant jusqu'à 5 atomes de carbone

. **n** vaut 3, 4 ou 5

. **p** vaut 1 ou 2

. **q** vaut 0 ou 1 et **p** + **q** vaut au plus 2

. et tel que le point de fusion soit supérieur à 50 °C.

Bien que tous ces produits A1 conviennent, si le matériau d'enregistrement sensible à la chaleur est formé à partir d'un substrat tel que du papier ou un film en résine synthétique, on utilise avantageusement les produits de l'invention ayant un point de fusion compris entre 50 et 220 °C. Parmi les produits de l'invention on utilise ceux dans lesquels $R_1$ et $R_2$ sont choisis parmi $CH_3$, Cl et H.

Ces produits peuvent être préparés par une condensation de FRIEDEL et CRAFTS (F et C) de :

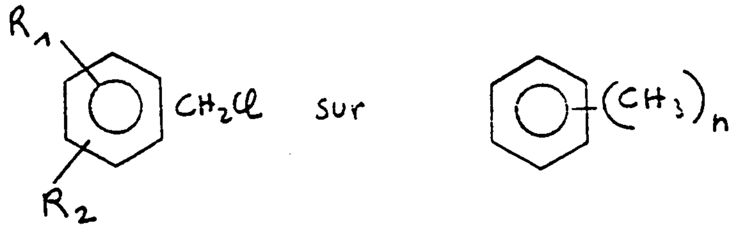

Le principe de cette réaction est connu. Le catalyseur de F et C est un halogénure ou un acide minéral. On utilise par exemple le chlorure ferrique, le trichlorure d'antimoine, le tétrachlorure de titane ou encore le chlorure d'aluminium. On peut aussi utiliser l'acide sulfurique ou des zéolites. Il suffit de mettre les deux réactifs en présence du catalyseur. Après réaction on peut distiller pour éliminer l'excédent éventuel de l'un ou des réactifs.

On peut aussi, après distillation du ou des réactifs en excès, procéder à l'élimination du catalyseur F et C par tout moyen connu tel que lavage à l'eau, neutralisation, séchage.

Dans le cas particulier des produits dans lesquels $R_1$ et $R_2$ sont $CH_3$ et n vaut 3 on peut préparer le chlorure $(CH_3)_2C_6H_3CH_2Cl$ in situ, c'est-à-dire qu'on part de $(CH_3)_3C_6H_3$ qu'on chlore partiellement par chloration radicalaire puis on ajoute dans le milieu de réaction qui est un mélange de $(CH_3)_2C_6H_3CH_2Cl$ et $(CH_3)_3C_6H_3$ un catalyseur de F et C. On effectue alors la condensation de F et C comme décrit précédemment. La chloration radicalaire des hydrocarbures aromatiques est connue. Une telle technique pour le toluène est décrite dans le brevet EP 136 230 dont le contenu est incorporé dans la présente demande. Il suffit en fin de réaction de récupérer les produits par distillation.

Certain des produits de formule A1 sont nouveaux. La présente invention concerne donc des produits de formule A2 :

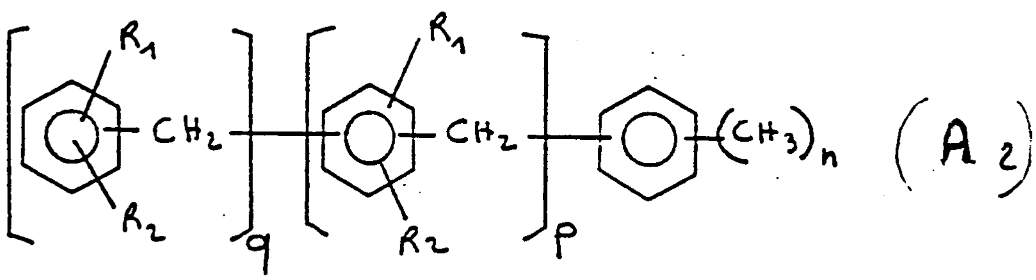

dans laquelle :

- . **$R_1$, $R_2$** identiques ou différents sont choisis parmi les halogènes NO2, CN, -OCH3, H ou un alkyle ayant jusqu'à 5 atomes de carbone.
- . **n** vaut 3, 4 ou 5
- . **p** vaut 1 ou 2
- . **q** vaut 0 ou 1 et **p + q** vaut au plus 2
- . tels que le point de fusion soit supérieur à 50 °C.
- . et les produits tels que

a/

$$q = 0$$
$$p = 1$$

$R_1 = CH_3$ en position ortho $R_2$ = halogène ou H

b/

$$q = 0$$
$$p = 1$$

$R_2$ = $CH_3$ en position ortho, $R_1$ = halogène ou H étant exclus.

On utilise avantageusement les produits dans les- quels **q** = O, **p** = 1 et **R**$_1$ et **R**$_2$ sont choisis parmi H et $CH_3$.

La fabrication de ces matériaux d'enregistrement est connue en soi.

**EXEMPLE 1 :**

Dans un réacteur muni d'une agitation rotative, d'une gaine thermométrique, d'un injecteur de chlore, on place :

3,33 moles de mésitylène (400 g) contenant 150 mg d'azobisisobutyronitrile (AIBN).

L'ensemble est porté à 150 ° C et on introduit 1 mole de chlore en 1 heure avec introduction simultanée de 350 mg d'AIBN en solution dans 0,67 mole de mésitylène (80 g).

Le milieu réactionnel est ensuite dégazé à l'azote et placé dans une ampoule de coulage. Il est introduit dans un réacteur muni d'une agitation rotative dans lequel on a placé 1,9 mole de mésitylène (228 g) et 2,2 g de chlorure ferrique anhydre. La température est de 140 ° C et la durée du coulage est de 1 heure. La réaction est poursuivie encore 2 h 30 à 150 ° C après introduction de 1,5 g supplémentaire de chlorure ferrique. Le milieu réactionnel après dégazage est ensuite soumis à une distillation avec une colonne d'environ 4 plateaux sous un vide de 10 mm de mercure pour éliminer le mésitylène non réagi. Le vide est ensuite abaissé à 1 mm de mercure. On obtient 90 g d'une fraction distillant à 151 ° C constituée par le composé :

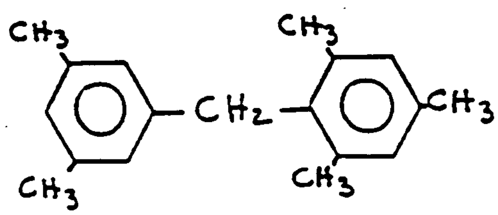

dont le point de fusion est de 66 ° C et la pureté d'après l'analyse chromatographique de 97,8 %. C'est un solide blanc, incolore à l'état fondu. La teneur en chlore résiduel est de 550 ppm.

**EXEMPLE 2 :**

Dans un réacteur muni d'une agitation rotative, d'un réfrigérant ascendant, d'une gaine thermométrique, d'un injecteur d'azote, on place 4 moles de durène (537 g) que l'on porte à 140 ° C. On ajoute 2,1 g de chlorure ferrique et on introduit par l'intermédiaire d'une ampoule de coulage 1,33 mole de chlorure de benzoyle (168 g) en 1 heure avec balayage d'azote. On ajoute 0,5 g de $FeCl_3$ et on laisse en réaction encore 3 heures à 140-150 ° C. L'acide chlorydrique dégagé a été recueilli dans un barboteur à eau et représente 96 % du théorique.

Le milieu réactionnel est ensuite soumis directement à une distillation avec une colonnne d'environ 4 plateaux sous un vide d'environ 15 mm de mercure. Après séparation du durène non réagi, on enlève la colonne à distiller, et on poursuit la distillation. Nous obtenons une fraction distillant à 184-185 ° C sous 15 mm de mercure constitué par le benzyldurène :

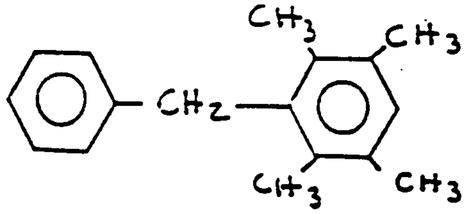

dont le poids de 200 g représente un rendement de 67 % par rapport au chlorure de benzyle engagé. La pureté d'après l'analyse chromatographique est de 98,2 %. Le point de fusion est de 55 ° C. C'est un

solide blanc, incolore à l'état fondu. Ce produit a une teneur en chlore inférieure à 20 ppm.

**EXEMPLE 3 :**

On effectue la condensation du chlorure de benzyle avec le pentaméthylbenzène avec un mode opératoire semblable à celui de l'exemple 2 en utilisant un rapport molaire chlorure de benzyle/pentaméthylbenzène = 1/5.

Nous obtenons après réaction et distillation une fraction distillant à 202-206°C (sous 10 mm de mercure de benzylpentaméthylbenzène :

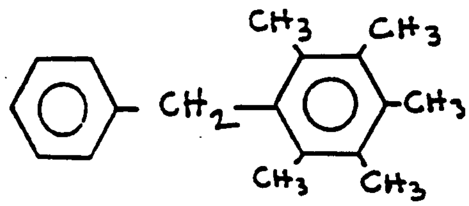

avec un rendement de 73 % par rapport au chlorure de benzyle engagé.

La pureté chromatographique est de 98,8 % et le point de fusion est de 112°C. C'est un solide blanc, incolore à l'état fondu. La teneur en chlore est inférieure à 20 ppm.

**EXEMPLE 4 :**

On effectue la condensation du chlorure de orthométhylbenzyle sur le durène selon un mode opératoire identique à celui de l'exemple 2 en utilisant un rapport molaire chlorure d'orthométhylbenzyle/durène = 1/5.

Nous obtenons après réaction et distillation une fraction distillant à 213-217°C sous 32 mm de mercure du produit de formule :

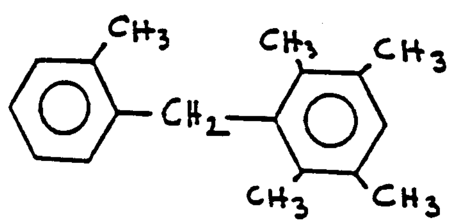

avec un rendement de 53 % par rapport au chlorure d'orthométhylbenzyl engagé.

La pureté chromatographique est de 98,1 % et le point de fusion de 98°C. C'est un solide blanc, incolore à l'état fondu.

**EXEMPLE 5 :**

Identique à l'exemple 4 mais en remplaçant le chlorure d'orthométhylbenzyl par le chlorure de paraméthylbenzyl. Nous obtenons après réaction et distillation une fraction distillant à 213-214°C sous 25 mm de mercure de :

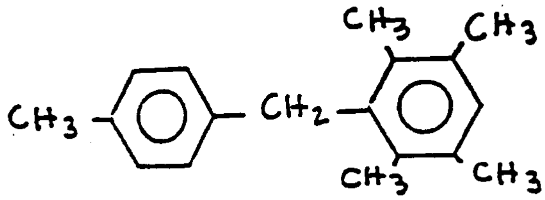

avec un rendement de 68 % par rapport au chlorure de paraméthylbenzyl engagé.

La pureté chromatographique est de 97,1 % et le point de fusion de 105°C.

**EXEMPLE 6 :**

Identique à l'exemple 2 mais en utilisant le chlorure de parachlorobenzyle, le rapport molaire chlorure de parachlorobenzyle/durène étant égal à 1/3.

Nous obtenons après réaction et distillation une fraction distillant vers 214-217 °C sous un vide de 16 mm de mercure de parachlorobenzyldurène :

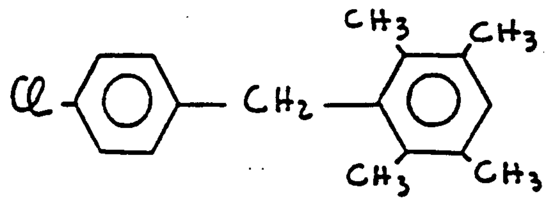

avec un rendement de 70 % par rapport au chlorure de paraxylyle engagé. La pureté chromatographique est de 99,3 % et le point de fusion de 102 °C. C'est un solide blanc incolore à l'état fondu.

La distillation a été poursuivie sur le résidu de distillation sous un vide de 6 mm de mercure. Nous avons obtenu une fraction distillant à 270-280 °C constituée par du bis(parachlorobenzyl)durène :

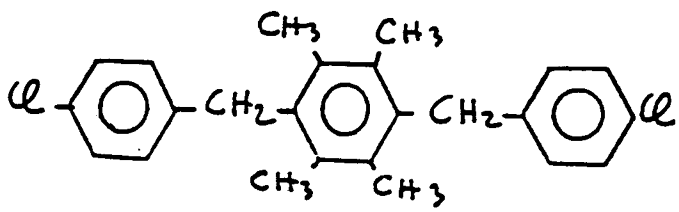

La pureté chromatographique est de 91,8 %(contient encore 3,6 % de parachlorobenzyldurène) et le point de fusion est de 210 °C.

**EXEMPLE 7 :**

Dans des conditions similaires à celles de l'exemple 2 on réalise la condensation de chlorure de benzyle avec le mésitylène dans un rapport molaire chlorure de benzyle/mésitylène égal à 1/2.

Nous obtenons après réaction et distillation une fraction distillant à 164-165 °C sous 12 mm de mercure de benzylmésitylène :

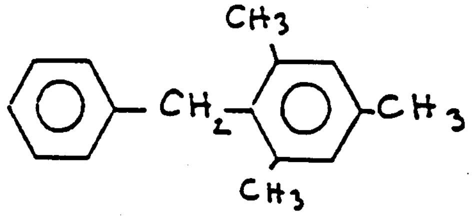

avec un rendement de 59 %. La pureté chromatographique est de 98,5 %. C'est un liquide à température ambiante.

La distillation a été poursuivie sur le résidu sous un vide de 12 mm de mercure et nous obtenons une fraction distillant à 245-246 °C constituée par du dibenzylmésitylène

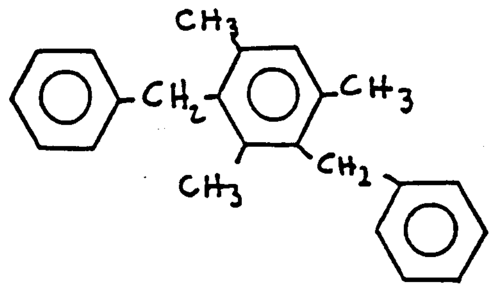

La pureté chromatographique est de 93,5 % et le point de fusion de 73 ° C.

**EXEMPLE 8 :**

Le broyage intime de 4 parties de benzylpentaméthylbenzène (BPB) obtenu dans l'Exemple 3, avec 1 partie de THA 108("color former" que l'on trouve sur le marché) et 2,5 parties de THA 70("color developper" que l'on trouve sur le marché) conduit à un seul pic en anaylse thermique comme l'indique la figure 1/2 et confirme que ce produit est utilisable comme sensibilisant dans la fabrication de papier thermique (FIGURE 1/2)

Le BPB, associé avec un "color former" et un "color developper" puis couché sur papier selon des techniques analogues à celles décrites dans les brevets NIPPON STEEL EP 164 417 ou WIGGINGS TEAPE EP 343 014 conduit à un papier thermosensible utilisable dans la télécopie.

**EXEMPLE 9 :**

Le broyage intime de 6 parties de dibenzylmésithylène (DBM) obtenu selon le mode opératoire de l'Exemple 7 (pureté 96 % F = 89 %) avec 1 partie de CF 51("color former" que l'on trouve sur le marché) et 2,5 parties de bisphénol A conduit à un mélange dont le thermogramme (Fig 2/2)confirme que ce produit est utilisable comme sensibilisant dans la fabrication de papier thermique (Figure 2/2).

Le DBM associé avec un "color former" et un "color developper" puis couché sur papier selon des techniques analogues à celles décrites dans les brevets NIPPON STEEL EP 164 417 ou WIGGINS TEAPE EP 343 014 conduit à un papier thermosensible utilisable dans la télécopie.

## Revendications

**1.** Matériaux d'enregistrement sensibles à la chaleur constitués d'un substrat ayant une couche contenant un précurseur de colorant et un développeur sensibles à la chaleur la dite couche contenant aussi un polyphénylméthane de formule A1

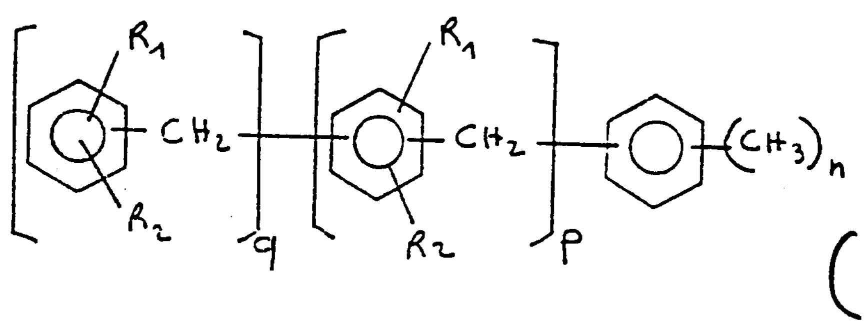

(A1)

dans laquelle :

$R_1$, $R_2$ identiques ou différents sont choisis parmi les halogènes, $NO_2$, CN, $OCH_3$, H ou un alkyle ayant jusqu'à 5 atomes de carbone

. **n** vaut 3, 4 ou 5

. **p** vaut 1 ou 2

. **q** vaut 0 ou 1 et **p** + **q** vaut au plus 2

. et tel que le point de fusion soit supérieur à 50 ° C.

**2.** Matériaux selon la revendication 1 caractérisés en ce que le point de fusion du polyphénylméthane est compris entre 50 et 220 ° C.

**3.** Matériaux selon la revendication 1 ou 2 caractérisés en ce que $R_1$ et $R_2$ sont choisis parmi $CH_3$, Cl et H.

**4.** Procédé de préparation des polyphénylméthanes tels que definis dans les revendications 1 à 3 caractérisé en ce qu'on effectue une condensation de FRIEDEL et CRAFTS de

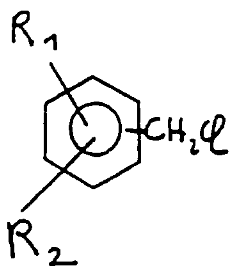

sur

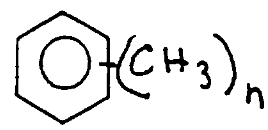

**5.** Procédé de préparation des polyphénylméthanes tels que definis dans les revendications 1 à 4 dans lesquels **$R_1$** et **$R_2$** sont $CH_3$ et **n** vaut 3 caractérisé en ce que dans une première étape on effectue une chloration radicalaire d'une partie de $(CH_3)_3C_6H_3$ puis dans une seconde étape on effectue une réaction de FRIEDEL et CRAFTS entre $(CH_3)_2C_6H_3CH_2Cl$ et $(CH_3)_3C_6H_3$.

**6.** Polyphénylméthanes de formule A2 :

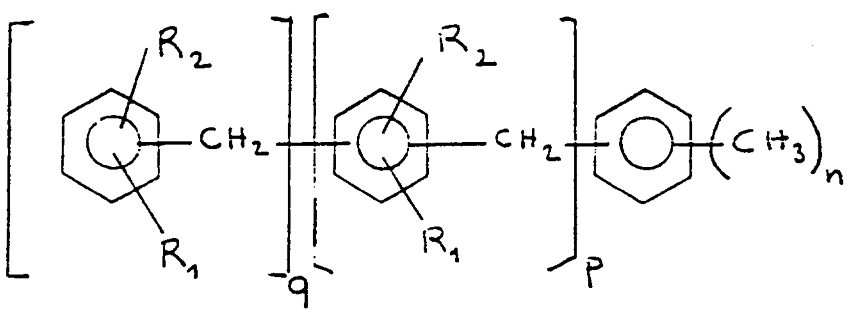

(A2)

dans laquelle :

. **$R_1$, $R_2$** identiques ou différents sont choisis parmi les halogènes NO2, CN, -OCH3, H ou un alkyle ayant jusqu'à 5 atomes de carbone.
. **n** vaut 3,4 ou 5
. **p** vaut 1 ou 2
. **q** vaut 0 ou 1 et **p** + **q** vaut au plus 2
. tels que le point de fusion soit supérieur à 50 ° C.
. et les produits tels que

a/

$$q = 0$$
$$p = 1$$

$R_1$ = $CH_3$ en position ortho, $R_2$ = halogène ou H

b/

$$q = 0$$
$$p = 1$$

$R_2$ = $CH_3$ en position ortho, $R_1$ = halogène ou H étant exclus.

## Claims

**1.** Heat-sensitive recording materials comprising a substrate having a layer containing a dyestuff precursor and a developer which are sensitive to heat, the said layer also containing a polyphenylmethane of the formula A1

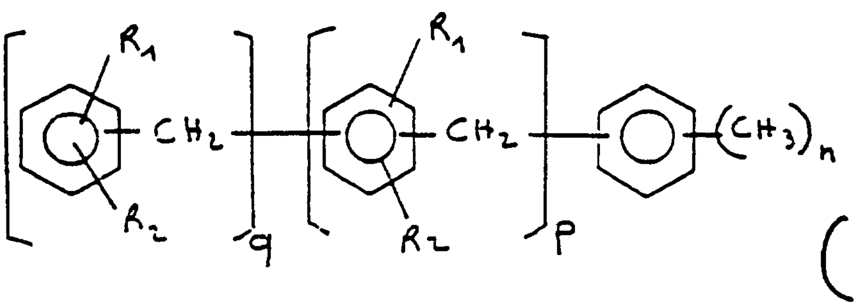

(A1)

in which :

$R_1$ and $R_2$ are identical or different and are chosen from halogens, $NO_2$, CN, $OCH_3$, H and an alkyl having up to 5 carbon atoms,

- n has the value 3, 4 or 5,
- p has the value 1 or 2,
- p has the value 0 or 1 and p + q has a value of no more than 2,
- and such that the melting point is higher than 50 °C.

**2.** Materials according to Claim 1, characterised in that the melting point of the polyphenylmethane is between 50 and 220 °C.

**3.** Materials according to Claim 1 or 2, characterised in that $R_1$ and $R_2$ are chosen from $CH_3$, Cl and H.

**4.** Process for the preparation of polyphenylmethanes as defined in Claims 1 to 3, characterised in that a Friedel-Crafts condensation of

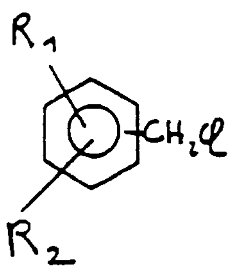

with

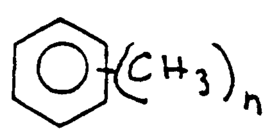

is carried out.

**5.** Process for the preparation of polyphenylmethanes as defined in claims 1 to 4 in which $R_1$ and $R_2$ are $CH_3$ and n has the value 3, characterised in that, in a first stage, a free radical chlorination is carried out on one portion of $(CH_3)_3C_6H_3$, and then, in a second stage, a Friedel-Crafts reaction is carried out between $(CH_3)_3C_6H_3CH_2Cl$ and $(CH_3)_3C_6H_3$.

**6.** Polyphenylmethanes of the formula A2 :

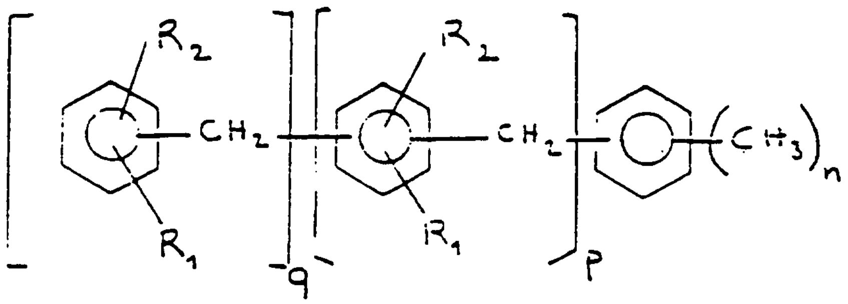

(A2)

in which:

- $R_1$ and $R_2$ are identical or different and are chosen from the halogens, $NO_2$, CN, $-OCH_3$, H and an alkyl having up to 5 carbon atoms,
- n has the value 3, 4 or 5,
- p has the value 1 or 2,
- q has the value 0 or 1 and p + q has a value of no more than 2,
- such that the melting point is higher than 50 °C,
- and excluding products where

a/

$$q = 0$$
$$p = 1$$

$R_1$ = $CH_3$ in the ortho-position and $R_2$ = halogen or H

b/

$$q = 0$$
$$p = 1$$

$R_2$ = $CH_3$ in the ortho-position and $R_1$ = halogen or H.

## Patentansprüche

**1.** Materialien zur thermographischen Aufzeichnung, bestehend aus einem Substrat mit einer einen Färbemittelvorläufer und einen hitzeempfindlichen Entwickler enthaltenden Schicht, die ferner ein Polyphenylmethan der Formel A1

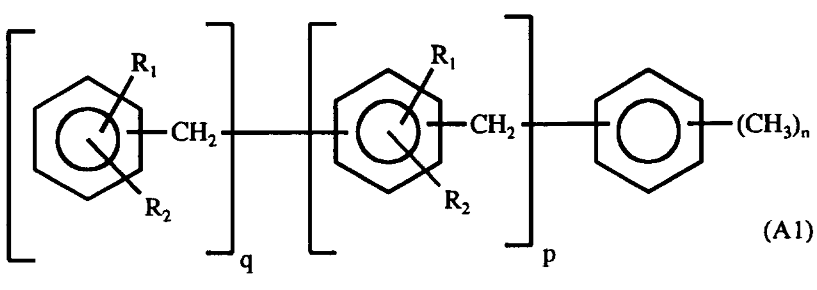

(A1)

enthält, in der:

- $R_1$ und $R_2$ gleich oder unterschiedlich sind, und aus Halogenatomen, $-NO_2$, -CN, $-OCH_3$, -H oder einem Alkylrest mit bis zu 5 Kohlenstoffatomen ausgewählt sind,
- n 3, 4 oder 5 ist,
- p 1 oder 2 ist,
- q 0 oder 1 ist und p + q höchstens 2 ist,
- wobei der Schmelzpunkt über 50 °C liegt.

**2.** Materialien nach Anspruch 1, dadurch gekennzeichnet, daß der Schmelzpunkt des Polyphenylmethans zwischen 50 und 220 °C liegt.

**3.** Materialien nach Anspruch 1 oder 2, dadurch gekennzeichnet, daß $R_1$ und $R_2$ aus $-CH_3$, -Cl und -H ausgewählt sind.

**4.** Verfahren zur Herstellung von Polyphenylmethanen nach einem der Ansprüche 1 bis 3, dadurch gekennzeichnet, daß man eine Friedel-Crafts-Kondensation von

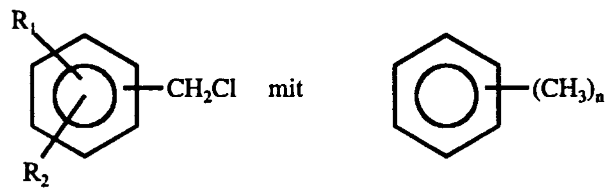

durchführt.

**5.** Verfahren zur Herstellung von Polyphenylmethanen nach einem der Ansprüche 1 bis 4, in denen $R_1$ und $R_2$ $-CH_3$ sind und n gleich 3 ist, dadurch gekennzeichnet, daß in einem ersten Schritt eine radikalische Chlorierung eines Teils des $(CH_3)_3C_6H_3$ durchgeführt wird und anschließend in einem zweiten Schritt eine Friedel-Crafts-Reaktion zwischen $(CH_3)_2C_6H_3CH_2Cl$ und $(CH_3)_3C_6H_3$ durchgeführt wird.

**6.** Polyphenylmethane der Formel A2:

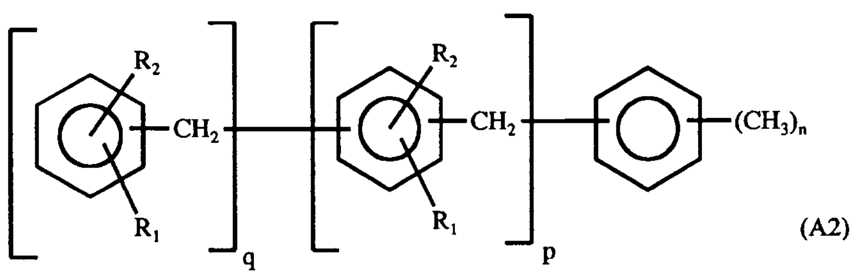

(A2)

in der:

- $R_1$ und $R_2$ gleich oder unterschiedlich sind und aus Halogenatomen, $-NO_2$, -CN, $OCH_3$, -H oder einem Alkylrest mit bis zu 5 Kohlenstoffatomen ausgewählt sind,
- n 3, 4 oder 5 ist,
- p 1 oder 2 ist,
- q 0 oder 1 und p + q höchstens 2 ist,
- wobei der Schmelzpunkt über 50 °C liegt,
- und die Produkte mit

a/

$$q = 0,$$
$$p = 1,$$

$R_1$ = $CH_3$ in der ortho-Position, $R_2$ = Halogenatom oder H, und

b/

$$q = 0,$$
$$p = 1,$$

$R_2$ = $CH_3$ in der ortho-Position, $R_1$ = Halogenatom oder H ausgeschlossen sind.

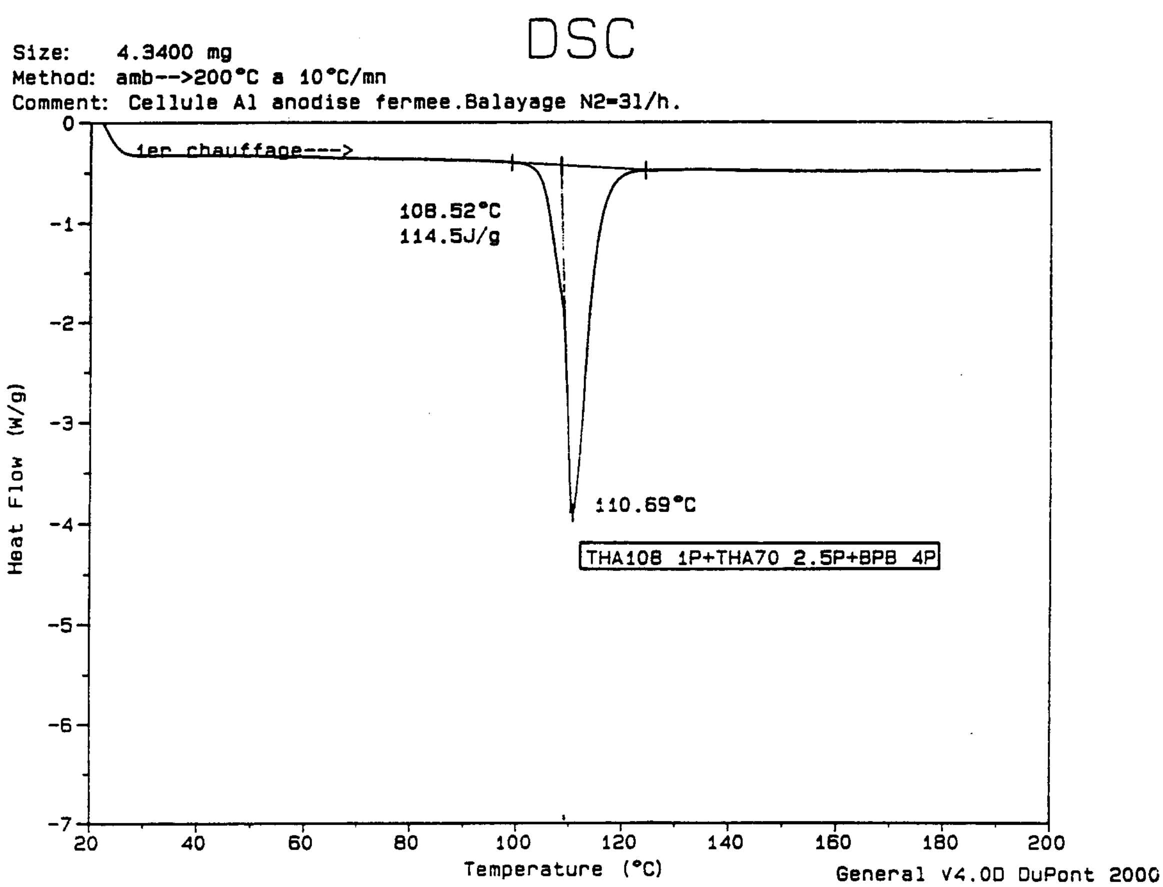
DSC
Size: 4.3400 mg
Method: amb-->200°C a 10°C/mn
Comment: Cellule Al anodise fermee.Balayage N2=31/h.
1er chauffage--->
108.52°C
114.5J/g
110.69°C
THA108 1P+THA70 2.5P+BPB 4P
Heat Flow (W/g)
0
-1
-2
-3
-4
-5
-6
-7
20
40
60
80
100
120
140
160
180
200
Temperature (°C)
General V4.0D DuPont 2000

FIGURE 1

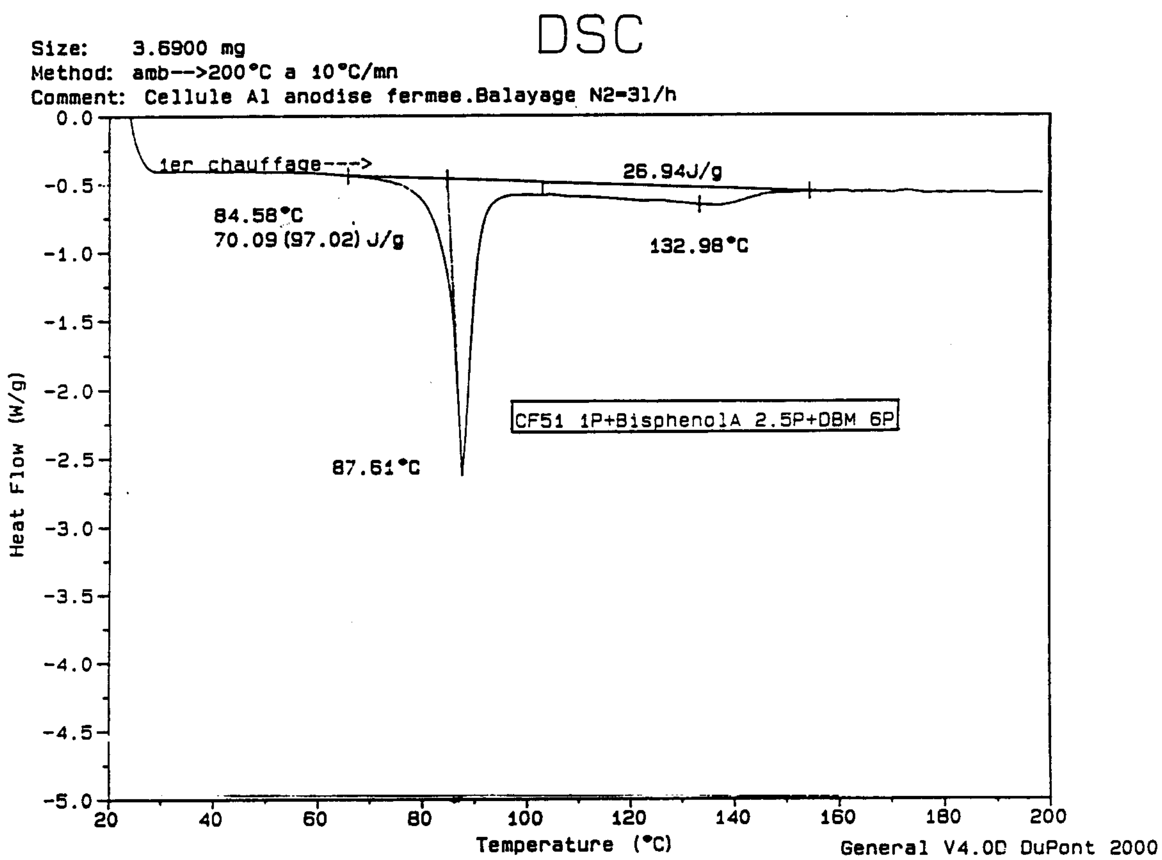

DSC
Size: 3.6900 mg
Method: amb-->200°C a 10°C/mn
Comment: Cellule Al anodise fermee.Balayage N2=31/h
1er chauffage--->
26.94J/g
84.58°C
70.09 (97.02) J/g
132.98°C
CF51 1P+BisphenolA 2.5P+DBM 6P
87.61°C
Heat Flow (W/g)
0.0
-0.5
-1.0
-1.5
-2.0
-2.5
-3.0
-3.5
-4.0
-4.5
-5.0
20
40
60
80
100
120
140
160
180
200
Temperature (°C)
General V4.0C DuPont 2000


FIGURE 2